# EUROPEAN PATENT APPLICATION

(11) **EP 4 714 267 A1**
(43) Date of publication of application: **25.03.2026**
(21) Application number: 24201324.1
(22) Date of filing: 19.09.2024
(51) Int. Cl.: A23G 3/34, A23G 3/36, A23G 3/48, A23G 4/06, A23G 4/12, A61K 9/68, A61K 47/12, A61K 47/22

(54) **A NICOTINE BASED GUM FORMULATION**

(71) Applicant: Mehta, Bharat, Sultanpur New Delhi 110030 (IN)
(72) Inventor: Mehta, Bharat, Sultanpur New Delhi 110030 (IN)
(74) Representative: HGF

(57) **Abstract**

The present invention is related to nicotine containing chewing gum, a method of manufacturing the chewing gum and its use as a nicotine replacement therapy without the common side effects such like hiccups, headaches, nausea and burning sensation of the throat. The present invention is particularly related to a nicotine based gum formulation comprising nicotine, a gelling agent, plasticizer, releasing agent, acidifier, acid regulating agent, and one or more pharmaceutical excipients. The nicotine based gum formulation can effectively deliver nicotine at a low pH-value.

## Description

### TECHNICAL FIELD

The presently disclosed subject matter relates to a nicotine containing chewing gum, a method of manufacturing the chewing gum and its use as a nicotine replacement therapy without the common side effects such like hiccups, headaches, nausea and burning sensation of the throat. The disclosed subject matter includes nicotine containing chewing gum which can effectively deliver nicotine at a low pH-value.

### BACKGROUND

Smoking behaviour is associated with serious health risks not only to the smoker, but also to the people around them exposed to passive smoke. Thus, experts from many years have advised to quit smoking. However, the smoker's addiction to nicotine, makes it difficult for quitting for most of the smokers. Many ways of nicotine administration have been employed in the efforts to help smokers quit their unhealthy habit. Several products employing oral or transdermal administration of nicotine are currently available in the market for smokers wanting to quit smoking such as chewing gums, inhalers, patches or mouth sprays.

As tobacco itself contains several other toxic compounds other than nicotine, nicotine substitution products are also relevant for individuals who consume their tobacco in other ways than by smoking. The main problem with tobacco smoking is its enormous implications on health. Today it is estimated that use of tobacco causes more than 8 million deaths per year. Even though today tobacco smoking is decreasing in many developed countries today there is a continuous need of creating awareness for quitting this unhealthy habit.

Nicotine is an addictive poisonous alkaloid C₁₀H₁₄N₂, derived from the tobacco plant. It is an organic compound and is the principal alkaloid of tobacco. Nicotine is the main addictive ingredient in the tobacco used in cigarettes, cigars, snuff and other nicotine-containing products. Nicotine is the world's second most used drug, after caffeine from coffee and tea. It is very addictive and thus characteristically display a strong tendency to relapse after having successfully abstained from smoking for a time. The administration of nicotine can provide satisfaction. When smoking a cigarette, nicotine is quickly absorbed into the smoker's blood and reaches the brain in around ten seconds after inhalation. The quick uptake of nicotine gives the consumer a rapid satisfaction, or a kick. The satisfaction of smoking a cigarette lasts during the smoking time and for a period of time thereafter. The poisonous, toxic, carcinogenic, and addictive nature of smoking has initiated efforts to search for methods, compositions and devices, which would help in breaking the habit of smoking cigarettes.

Several methods and means have been described for reducing the desire of a subject to use tobacco, which comprises the step of administering to the subject nicotine or a derivative thereof as described in few of the patents applications. The applications are US4967773A (Nicotine containing lozenge), US20240000713 (Water-soluble nicotine tablet), GB2299756 (Pastilles containing nicotine), US5810018A (oral nicotine spray), US20100004294 (Stable lozenge containing nicotine).

Thus, there are prior arts available for nicotine containing various formulations for reducing the nicotine addiction. The major formulation available in the market is a gum base formulation or solid formulation like lozenges. But there are various problems in the prior art composition as nicotine is retained in the gum due to incomplete chewing resulting in low systemic bioavailability. The chewing of gums or lozenges mentioned in the prior art leads to all of a sudden release of nicotine in buccal cavity which in turn causes nausea and burning sensation. Moreover, accidental swallowing results in sudden release of nicotine in GI tract causing nausea and burning sensation.

Soft gum products with a pH of 5.0 or higher tend to be less enjoyable to consume due to a pronounced nicotine release, which can lead to strong side effects such as hiccups, headaches, nausea, and a burning throat. In contrast, gums with a pH below 5.0 experience reduced nicotine release and fewer side effects. Specifically, gums with a pH ranging from 3.5 to 3.9 not only lower the nicotine impact but also improve the masking of both nicotine and the product's taste. This improvement is particularly noticeable in gums with natural plant flavors like peppermint, orange-lemon, and blueberry. For gums with more neutral flavors, such as those mimicking dried or processed plant parts like coffee and cinnamon, the best masking of nicotine occurs at a pH between 4.0 and 4.5.

Though there are various nicotine containing formulations available in the market, there is a need to develop a formulation overcoming all the shortcomings of the available formulations. It is an aim of the present invention to provide a formulation that maximizes buccal absorption of nicotine. By providing a low pH-value of soft gum, this allows for a gradual pH-value increase as the formulation stays in the mouth which, in turn, allows for a controlled, gradual nicotine release. Also, the present formulation overcomes the associated symptoms of nicotine release such as nausea, hiccups or burning sensation by providing formulation with pH below 5.0. Furthermore, the oral formulation of the present invention provides release of nicotine in a controlled manner over a prolonged period of time without unpleasant mouth feel and taste.

### SUMMARY

Disclosed is a chewing gum formulation and process preparation thereof. The chewing gum of the present invention is adapted to be held in the user's mouth to release the active ingredient into the oral cavity in a manner determined by the user.

A first aspect of the invention provides a nicotine based gum formulation comprising nicotine, a gelling agent, plasticizer, releasing agent, acidifier, acid regulating agent, and one or more pharmaceutical excipients.

The another objective of the present invention is to provide a nicotine based gum formulation comprising nicotine, a gelling agent, plasticizer, releasing agent, acidifier, acid regulating agent, flavouring agent, sweetener, thickening agent and colouring agent.

The formulation can be used by smokers either as a substitute for cigarettes, or as an aid in smoking cessation or as a nicotine substitute in smoking cessation therapy.

The formulation can maximize buccal absorption of nicotine and release the active ingredient in a controlled manner over a prolonged period of time without an unpleasant taste and mouth feel.

As per another aspect, the proposed chewing gum contains nicotine formulated with flavours that are pleasant for consumers like lemon-orange, cinnamon, blueberry, mint, coffee, banana, vanilla, chocolate it improves user perception significantly and reduces mouth and throat irritation and removes unpleasant mouth feel and taste.

A second aspect of the invention provides a nicotine based gum formulation according to the first aspect for use in the treatment of nicotine dependence.

A third aspect of the invention provides a process of preparing a nicotine based gum formulation according to the first aspect comprising the following steps:
a) Weighing plasticizer and water in a reactor;
b) Adding gelling agent and releasing agent in mixture of step (a);
c) Adding nicotine active in the mixture of step (b);
d) Adding adequate quantities of sweetener, acidifiers, acid regulating agent, thickening agents, flavouring agents and colouring agents in the mixture of step (c) obtaining a mass;
e) Collecting the mass of step (d) in a container followed by cooling and solidification;
f) Transferring the solidified mass of step of step (e) into a melter to obtain melted mass;
g) Passing the melted mass of step (f) through an injector into the preformed cavities followed by cooling and blister packaging.

### DETAILED DESCRIPTION OF THE INVENTION

The detailed description set forth below is intended as a description of exemplary embodiments and is not intended to represent the only forms in which the exemplary embodiments may be constructed and/or utilized. The description sets forth the functions and the sequence of steps for constructing and/or operating the exemplary embodiments. However, it is to be understood that the same or equivalent functions and sequences which may be accomplished by different exemplary methods are also intended to be encompassed within the spirit and scope of the invention.

As defined herein, all scientific and technical terms used herein have the same meaning as commonly understood by one of ordinary skill in the art to which the invention belongs.

Although any process and materials similar or equivalent to those described herein can be used in the practice or testing, the preferred methods and materials are now described.

As stated in the present invention herein, the singular forms "a," "an" and "the" specifically also encompass the plural forms of the terms to which they refer, unless the content clearly dictates otherwise. The term "about" is used herein to mean approximately, in the region of, roughly, or around.

As stated herein, that it follows in a transitional phrase or in the body of a claim, the terms "comprise(s)" and "comprising" are to be interpreted as having an open ended meaning. That is, the terms are to be interpreted synonymously with the phrases "having at least" or "including at least". When used in the context of a process, the term "comprising" means that the process includes at least the recited steps, but may include additional steps. When used in the context of a composition, the term "comprising" means that the composition includes at least the recited features or components, but may also include additional features or components.

As mentioned herein the body of the invention the "gums are not coated formulation" includes soft gum, pastilles, soft pastilles, chewing pastilles, as well as gelled, dimensionally stable soft gel products and hard gum products.

The term "chewing gum" is a novel drug delivery system containing gum base with pharmacologically active ingredient and intended to use for systemic absorption through oral mucosa.

In an aspect of the present invention, there is provided a nicotine based gum formulation comprising nicotine, a gelling agent, plasticizer, releasing agent, acidifier, acid regulating agent, and one or more pharmaceutical excipients.

In an embodiment, there is provided a nicotine based gum formulation comprising nicotine in an amount of about 0.1 - 0.2 wt% , a gelling agent in an amount of about 20 wt%, plasticizers in an amount of about 50 wt%, releasing agent in an amount of about 6 - 7 wt%, acidifier in an amount of about 3-4 wt%, acid regulating agent in an amount of about 0.35-0.45 wt%, and one or more pharmaceutical excipients.

In an embodiment, the nicotine based gum formulation comprises nicotine, gelatin, glycerin, lecithin, ascorbic acid, citric acid, trisodium citrate, and one or more pharmaceutical excipients.

In embodiments, the nicotine based gum formulation is provided as a unit dose of a soft gum. In embodiments, the unit dose may be about 800mg to about 1600mg of a soft gum. In embodiments, the unit dose may be about 1000mg to about 1400mg of a soft gum. In embodiments, the unit dose may be about 1200mg of a soft gum.

As per one embodiment of the present invention, nicotine is selected from group consisting of nicotine polacrilex, nicotine resinate and nicotine lactate and combinations thereof.

In a preferred embodiment, the nicotine is nicotine polacrilex.

In an embodiment, the nicotine based gum formulation said nicotine comprises from about 0.01% to about 1% of the mass of the soft gum. In an embodiment, the nicotine based gum formulation said nicotine comprises from about 0.1% to about 0.3% of the mass of the soft gum. In an embodiment, the nicotine based gum formulation said nicotine comprises about 0.167% to about 0.292% of the mass of the soft gum.

In an embodiment, the nicotine based gum formulation comprises 1 mg to 10 mg of nicotine per unit dose.

In an embodiment, the nicotine based gum formulation comprises 1 mg to 5 mg of nicotine per unit dose. In an embodiment, the nicotine based gum formulation comprises 2 mg to 4 mg of nicotine per unit dose. In an embodiment, the nicotine based gum formulation comprises about 2 mg of nicotine per unit dose. In an embodiment, the nicotine based gum formulation comprises about 3.5 mg of nicotine per unit dose.

In an embodiment of the present invention, the gelling agent is selected from tragacanth, pectin, starch, carbomer, sodium alginate, gelatin, cellulose derivatives, and polyvinyl alcohol clays.

In a preferred embodiment, the gelling agent is gelatin.

In an embodiment, the nicotine based gum formulation said gelling agent comprises about 15% to about 25% of the mass of the soft gum.

In an embodiment, the nicotine based gum formulation comprises 100 mg to 500 mg of gelling agent per unit dose.

In an embodiment, the nicotine based gum formulation comprises 100 mg to 250 mg of gelling agent per unit dose.

As per one embodiment of the present invention, plasticizer is selected from vegetable oils, hydrogenated vegetable oils, triacetin, glycerin, propylene glycol.

In a preferred embodiment, the plasticizer is glycerin.

In an embodiment, the nicotine based gum formulation said plasticizer comprises about 40% to about 70% of the mass of the soft gum.

In an embodiment, the nicotine based gum formulation comprises 100 mg to 1000 mg of plasticizer per unit dose.

In an embodiment, the nicotine based gum formulation comprises 500 mg to 1000 mg of plasticizer per unit dose.

In an preferred embodiment,

In an embodiment of the present invention, the releasing agent is selected from oil, lecithin, lanolin, mono-glycerides and di-glycerides, stearic acid, sodium stearate, glycerine, potassium stearate and glycerol triacetate.

In a preferred embodiment, the releasing agent is lecithin.

In an embodiment, the nicotine based gum formulation said releasing agent comprises about 5.0% to about 10% of the mass of the soft gum.

In an embodiment, the nicotine based gum formulation comprises 10 mg to 100 mg of releasing agent per unit dose.

In an embodiment, the nicotine based gum formulation comprises 50 mg to 100 mg of releasing agent per unit dose.

As per one embodiment of the present invention, acidifier is selected from group consisting of ascorbic acid, calcium ascorbate, citric acid and malic acid and combinations thereof.

In a preferred embodiment, the acidifier is combination of ascorbic acid and citric acid. Ascorbic acid used in the preparation of soft gums acts as an anti-oxidation agent. Citric acid meanwhile acts as a support for masking nicotine, supporting better taste and decreasing the pH-value.

In an embodiment, the nicotine based gum formulation said acidifier comprises about 1.0% to about 5.0% of the mass of the soft gum.

In an embodiment, the nicotine based gum formulation comprises 10 mg to 100 mg of acidifier per unit dose.

In an embodiment, the nicotine based gum formulation comprises 10 mg to 60 mg of acidifier per unit dose.

As per one embodiment of the present invention, acid regulating agent is selected from group consisting of sodium salts of water-soluble organic acids.

In a preferred embodiment, the acid regulating agent is sodium citrate. Sodium citrate used in the preparation of soft gums acts as a buffer salt to adjust the pH-value of the soft gum.

In an embodiment, the nicotine based gum formulation said acid regulating agent comprises about 0.0% to about 0.5% of the mass of the soft gum.

In an embodiment, the nicotine based gum formulation comprises 10 mg to 100 mg of acid regulating agent per unit dose.

In an embodiment, the nicotine based gum formulation comprises 10 mg to 60 mg of acid regulating agent per unit dose.

As per one embodiment of the present invention, one or more pharmaceutically acceptable excipients can be selected from flavouring agent, sweetener, thickening agent, colouring agent or combinations thereof.

As per one embodiment of the present invention, flavoring agents include, but are not limited to menthol, vanillin, cinnamon, peppermint, lemon, mint, coffee, strawberry, banana, pineapple, orange, raspberry and combinations thereof.

In an embodiment, the nicotine based gum formulation said flavoring agents comprises about 0.5% to 6.0% of the mass of the soft gum.

In an embodiment, the nicotine based gum formulation comprises 1 mg to 50 mg of flavoring agents per unit dose.

In an embodiment, the nicotine based gum formulation comprises 10 mg to 40 mg of flavoring agents per unit dose.

As per one embodiment of the present invention, sweetener can be selected from the group consisting of stevia, sucralose and sucrose and combinations thereof.

In an embodiment, the nicotine based gum formulation said sweetener comprises about 0.5% to about 1.0% of the mass of the soft gum.

In an embodiment, the nicotine based gum formulation comprises 0.5 mg to 15 mg of sweetener per unit dose.

In an embodiment, the nicotine based gum formulation comprises 1 mg to 10 mg of sweetener per unit dose.

As per one embodiment of the present invention thickening agent can be selected from the group consisting of guar gum, locust bean gum, inulin, gum arabic and carrageenan and combinations thereof.

In a preferred embodiment, the thickening agent is combination of guar gum and inulin.

In an embodiment, the nicotine based gum formulation said thickening agent comprises about 0.25% to about 5.00% of the mass of the soft gum.

In an embodiment, the nicotine based gum formulation comprises 1 mg to 50 mg of thickening agent per unit dose.

In an embodiment, the nicotine based gum formulation comprises 1 mg to 35 mg of thickening agent per unit dose.

In an embodiment of the present invention thickening agent can be selected from the group consisting of plant extracts or natural dyes.

In an embodiment, the nicotine based gum formulation water comprises about 5% to about 15% of the mass of the soft gum.

In an embodiment, the nicotine based gum formulation are free of preservatives.

In an embodiment, the pH of the nicotine based gum formulation is determined at 20-30 deg C by using a pH meter by dissolving and sonicating the 1200mg of gum for 10 to 20 minutes in 20 ml HPLC water.

As per one embodiment of the present invention, the pH-value of the soft gum is below pH 5.

In a preferred embodiment, the pH-value of the soft gum in the range of pH 3.0 to pH 4.8.

As per one embodiment of the present invention, said soft gum being capable of being dissolved in the buccal cavity in about 5 to about 15 minutes, depending on the user's sucking pattern.

### Treatment of Nicotine Dependence

The second aspect of the invention provides a nicotine based gum formulation according to the first aspect for use in the treatment of nicotine dependence.

As per one embodiment of the present invention, the treatment comprises use of one piece of gum every 1-2 hours for the first 6 weeks.

As per one embodiment of the present invention, the treatment comprises use of 1 piece every 2-4 hours, and then 1 piece every 4-8 hours, following 6 weeks of use.

As per one embodiment of the present invention, nicotine gum base formulation helps in reducing the side effects of nicotine like hiccups, headaches, nausea and burning sensation of the throat.

As per one embodiment of the present invention, nicotine gum base formulation is used as a nicotine replacement product for smokers or vapours.

### Process of Preparation

The third aspect of the invention provides a process of preparing a nicotine based gum formulation according to the first aspect of the invention comprising the steps of:
a) Weighing plasticizer and water in a reactor;
b) Adding gelling agent and releasing agent in mixture of step (a);
c) Adding nicotine active in the mixture of step (b);
d) Adding adequate quantities of sweetener, acidifiers, acid regulating agent, thickening agents, flavouring agents and colouring agents in the mixture of step (c) obtaining a mass;
e) Collecting the mass of step (d) in a container followed by cooling and solidification;
f) Transferring the solidified mass of step of step (e) into a melter to obtain melted mass;
g) Passing the melted mass of step (f) through an injector into the preformed cavities followed by cooling and blister packaging.

### EXAMPLES

### Example 1:

Each soft gum (1200 mg) contains:

**Table 1: Nicotine based gum formulation 1**

| **Ingredients** | **Quantity (in mg)** |
|---|---|
| Nicotine from Nicotine polacrilex | 2.0 mg |
| Gelatin (bloom strength 250) | 243.75 mg |
| Glycerin | 632.53 mg |
| Water | 98.72 mg |
| Lecithin | 80.0 mg |
| Ascorbic acid | 20.00 mg |
| Citric acid | 20.00 mg |
| Tri-sodium citrate | 5.00 mg |
| Sucralose | 4.50 mg |
| Steviol glycosides | 3.50 mg |
| Menthol | 2.00 mg |
| Plant extract (for coloring) | 5.00 mg |
| Inulin | 20.00 mg |
| Guar gum | 5.00 mg |
| Flavor (flavor oils) | 20.00 mg |
| Flavor | 30.00 mg |

### Procedure:

a) Weighing glycerin and water in a reactor;
b) Adding gelatin and lecithin in mixture of step (a);
c) Adding nicotine active in the mixture of step (b);
d) Adding adequate quantities of sucralose, ascorbic acid, citric acid, Cirtic acid, Tri-sodium citrate, guar gum, inulin, flavouring agents and colouring agents in the mixture of step (c) obtaining a mass;
e) Collecting the mass of step (d) in a container followed by cooling and solidification;
f) Transferring the solidified mass of step of step (e) into a melter to obtain melted mass;
g) Passing the melted mass of step (f) through an injector into the preformed cavities followed by cooling and blister packaging.

### Example 2:

Each soft gum (1200 mg) contains:

**Table 2: Nicotine based gum formulation 2**

| **Ingredients** | **Quantity** |
|---|---|
| Nicotine from Nicotine polacrilex | 3.5 mg |
| Gelatin (bloom strength 250) | 241.73 mg |
| Glycerin | 627.28 mg |
| Water | 97.90 mg |
| Lecithin | 80.0 mg |
| Ascorbic acid | 20.00 mg |
| Citric acid | 20.00 mg |
| Tri-sodium citrate | 5.00 mg |
| Sucralose | 4.80 mg |
| Steviol glycosides | 3.80 mg |
| Menthol | 2.00 mg |
| Plant extract (for coloring) | 5.00 mg |
| Inulin | 20.00 mg |
| Guar gum | 5.00 mg |
| Flavor (flavor oils) | 20.00 mg |
| Flavor | 30.00 mg |

### Procedure:

As per example 1 with table 2 excipients

### Example 3:

Each soft gum (1200 mg) contains:

**Table 3: Nicotine based gum formulation 3**

| **Ingredients** | **Quantity** |
|---|---|
| Nicotine from Nicotine polacrilex | 2.0 mg |
| Gelatin (bloom strength 250) | 206.10 mg |
| Glycerin | 713.11 mg |
| Water | 111.29 mg |
| Lecithin | 80.0 mg |
| Ascorbic acid | 20.00 mg |
| Sucralose | 4.50 mg |
| Steviol glycosides | 3.50 mg |
| Plant extract (for coloring) | 12.00 mg |
| Inulin | 3.00 mg |
| Guar gum | 1.5 mg |
| Flavor | 35.00 mg |

### Procedure:

As per example 1 with table 3 excipients.

## Claims

1. A nicotine based gum formulation comprising nicotine, a gelling agent, plasticizer, releasing agent, acidifier, acid regulating agent, and one or more pharmaceutical excipients.

2. The nicotine based gum formulation of claim 1 comprising nicotine in an amount of about 0.1 - 0.2 wt% , a gelling agent in an amount of about 20 wt%, plasticizers in an amount of about 50 wt%, releasing agent in an amount of about 6 - 7 wt%, acidifier in an amount of about 3-4 wt%, acid regulating agent in an amount of about 0.35-0.45 wt%, and one or more pharmaceutical excipients.

3. The nicotine based gum formulation as claimed in claim 1 or claim 2, wherein the said nicotine is selected from group consisting of nicotine polacrilex, nicotine resinate and nicotine lactate and combinations thereof.

4. The nicotine based gum formulation as claimed in any preceding claim, wherein the said gelling agent is selected from tragacanth, pectin, starch, carbomer, sodium alginate, gelatin, cellulose derivatives, and polyvinyl alcohol clays.

5. The nicotine based gum formulation as claimed in any preceding claim, wherein the said plasticizer is selected from lecithin, lanolin, glycerides, stearic acid, sodium stearate, glycerine, potassium stearate and glycerol triacetate.

6. The nicotine based gum formulation as claimed in any preceding claim, wherein the said releasing agent is selected from oil, lecithin, lanolin, mono-glycerides and di-glycerides, stearic acid, sodium stearate, glycerine, potassium stearate and glycerol triacetate.

7. The nicotine based gum formulation as claimed in any preceding claim, wherein the said acidifier is selected from group consisting of ascorbic acid, calcium ascorbate, citric acid and malic acid and combinations thereof.

8. The nicotine based gum formulation as claimed in any preceding claim, wherein the said acid regulating agent is selected from group consisting of sodium salts of water-soluble organic acids.

9. The nicotine based gum formulation as claimed in any preceding claim, wherein the said one or more pharmaceutical excipients can be selected from flavouring agent, sweetener, thickening agent, colouring agent or combinations thereof.

10. The the nicotine based gum formulation of claim 1, wherein the formulation comprises nicotine, gelatin, glycerin, lecithin, ascorbic acid, citric acid, trisodium citrate, and one or more pharmaceutical excipients.

11. The nicotine based gum formulation as claimed in any preceding claim for use in the treatment of nicotine dependence.

12. A process for preparing a nicotine based gum formulation, the process comprising the following steps:
a) Weighing plasticizer and water in a reactor;
b) Adding gelling agent and releasing agent in mixture of step (a);
c) Adding nicotine active in the mixture of step (b);
d) Adding adequate quantities of sweetener, acidifiers, acid regulating agent, thickening agents, flavouring agents and colouring agents in the mixture of step (c) obtaining a mass;
e) Collecting the mass of step (d) in a container followed by cooling and solidification;
f) Transferring the solidified mass of step of step (e) into a melter to obtain melted mass;
g) Passing the melted mass of step (f) through an injector into the preformed cavities followed by cooling and blister packaging.
